# FASCICULE DE BREVET EUROPEEN

(11) **EP 4 340 782 B1**
(45) Date de publication et mention de la délivrance du brevet: **31.12.2025**
(21) Numéro de dépôt: 22729552.4
(22) Date de dépôt: 16.05.2022
(51) Int. Cl.: A61F 2/50, A61B 5/107, A61B 5/00, G06T 17/00, A61F 2/76

(54) **DISPOSITIF ET MÉTHODE POUR LA PRISE D'EMPREINTES PAR NUMÉRISATION POUR LA GÉNÉRATION D'UN MODÈLE 3D D'EMBOÎTURE DE PROTHÈSE FÉMORALE**
VORRICHTUNG UND VERFAHREN ZUR ERFASSUNG DIGITALISIERTER ABDRÜCKE ZUR ERZEUGUNG EINES 3D-MODELLS EINER FASSUNG FÜR EINE OBERSCHENKELPROTHESE
DEVICE AND METHOD FOR ACQUIRING DIGITIZED IMPRESSIONS IN ORDER TO GENERATE A 3D MODEL OF A SOCKET FOR AN ABOVE-KNEE PROSTHESIS

(30) Priorité: 21.05.2021 FR 2105336
(43) Date de publication de la demande: 27.03.2024
(73) Titulaire: Vytruve, 35510 Cesson-Sévigné (FR)
(72) Inventeur: CALVIER, Erwan, 35700 RENNES (FR)
(74) Mandataire: Cabinet Le Guen Maillet
(86) Numéro de dépôt international: PCT/EP2022/063208
(87) Numéro de publication internationale: WO 2022/243255

(56) Documents cités:
- US-A- 4 988 360
- US-A1- 2010 115 757
- US-B2- 10 179 056

## Description

La présente invention concerne un dispositif d'aide à la prise d'empreintes par numérisation de la partie restante d'un membre amputé d'un patient pour la génération d'un modèle 3D d'une emboîture de prothèse fémorale, ceci en vue de la conception assistée par ordinateur de la prothèse fémorale.

La méthode de fabrication de prothèses pour des membres après amputation transfémorale comprend plusieurs étapes dont une étape de prise d'empreintes incluant la détermination de la forme du membre amputé, le repérage de points anatomiques importants tels que la position de proéminences osseuses, en particulier de l'ischion, ainsi que la compression nécessaire des masses molles. La méthode traditionnelle consiste à réaliser un moulage de la partie restante du membre amputé à l'aide de bandes de plâtre et à repérer manuellement et marquer sur le plâtre les parties anatomiques importantes.

Cette méthode est longue et fastidieuse, nécessite un certain savoir-faire et requiert parfois la présence de plusieurs orthoprothésistes. Le document US 4 988 360 A montre un dispositif selon le préambule de la revendication 1.

Le but de la présente invention est de proposer une méthode sûre, efficace et reproductible, pour la prise d'empreintes par numérisation et la conception d'un modèle 3D d'une emboîture de prothèse fémorale en vue de sa conception assistée par ordinateur, ainsi qu'un dispositif pour la mise en œuvre de ladite méthode.

A cet effet, l'invention concerne une méthode de génération d'un modèle 3D d'une emboîture de prothèse fémorale pour un patient en vue de sa conception assistée par ordinateur, suivant la revendication 13.

La méthode selon l'invention permet d'obtenir, par voie numérique, les données ou empreintes nécessaires à la génération d'un modèle 3D d'une emboîture de prothèse fémorale, ces données ou empreintes incluant :
- la position de l'ischion du patient et,
- la compression des masses molles.

Ainsi, les première et seconde étapes consistent à procéder à des marquages, sur le membre amputé, puis à obtenir un premier modèle numérique sur lequel le contour du membre et les marquages sont visibles. Ces marquages permettront de superposer le premier et le second modèles numériques 3D obtenus ultérieurement.

Les troisième et quatrième étapes consistent à installer le dispositif selon l'invention sur le membre amputé. Pour ce faire, le dispositif selon l'invention est installé sur le membre de manière à ce que l'une des languettes transparentes soit appliquée contre la face postérieure du membre, au-dessous de l'ischion. Plus spécifiquement, le bord supérieur que présente la languette est appliqué juste en dessous du bord antérieur de l'ischion. L'ischion se retrouve ainsi en appui contre cette languette lorsque le patient se trouve debout. La seconde languette peut quant à elle être appliquée par exemple contre la face extérieure ou intérieure du membre.

Une fois le dispositif mis en place et les languettes positionnées, celles-ci sont serrées contre le membre pour comprimer les masses molles, selon une intensité de serrage adaptée à chaque patient. La compression exercée au niveau des bords que présentent les languettes permet d'obtenir des zones de compression.

Lors de l'étape ultérieure, un second modèle numérique 3D est obtenu, incluant la position de l'ischion et les zones de compression des masses molles.

Les premier et second modèles numériques 3D sont ensuite superposés en prenant comme point de repère, pour cet alignement, les marquages réalisés lors de la première étape, ceux-ci étant visibles sur l'ensemble des modèles numériques 3D. Le premier modèle numérique 3D contient la forme du membre amputé. Le second modèle numérique 3D contient la position de l'ischion et les zones déterminant la compression des masses molles. Il est alors procédé à un traitement adéquat de manière à reconstituer, à partir des deux modèles numériques 3D, un modèle numérique 3D permettant la conception assistée par ordinateur d'une emboîture de prothèse fémorale.

Selon un mode de réalisation de l'invention, la méthode comprend, de plus, une étape de réglage de flexum de la hanche dudit patient, le dispositif comportant pour ce faire un appui au sol et des moyens de réglage de la position relative desdites languettes solidaires de la base par rapport audit appui au sol, ainsi que de l'angle antéro postérieur formé entre eux.

Lors de cette étape, l'orthoprothésiste positionne le patient debout sur le sol et procède aux réglages du dispositif, à l'aide de moyens de réglage prévus à cet effet, en respectant l'angulation naturelle de son flexum de hanche pour que le patient soit en appui de manière stable au sol.

L'invention concerne encore un dispositif d'aide à la prise d'empreintes par numérisation de la partie restante d'un membre amputé d'un patient pour la génération d'un modèle 3D d'une emboîture de prothèse fémorale, selon la revendication 1.

Selon un mode de réalisation préféré de l'invention, le dispositif comprend de plus :
- un appui au sol,
- un moyen de liaison entre ledit appui au sol et ladite base.

Avantageusement, le moyen de liaison comporte des moyens de réglage de la position relative desdites languettes solidaires de la base par rapport audit appui au sol ainsi que de l'angle antéro postérieur formé entre eux.

Préférentiellement, lesdits moyens de réglage comprennent une glissière concave dont la concavité est tournée vers la base ainsi qu'un guide que présente la base, ledit guide étant apte à coulisser le long de ladite glissière.

Avantageusement, ledit appui au sol comporte un piètement et un mat réglable en hauteur.

Préférentiellement, ledit piètement prend la forme d'un pied humain.

Préférentiellement encore, ledit appui au sol comprend, de plus, une liaison articulée, préférentiellement un pivot.

Selon une caractéristique de l'invention, la première des languettes présente un bord supérieur qui s'évase en direction de l'extérieur.

Selon une autre caractéristique de l'invention, la première languette fait saillie depuis une première portion du bord supérieur que présente la base, la seconde languette fait saillie depuis une seconde portion du bord supérieur adjacente à la première portion. Avantageusement, le dispositif comporte une troisième languette qui fait saillie d'une troisième portion du bord supérieur, préférentiellement en vis-à-vis de la deuxième portion.

Avantageusement encore, le dispositif comporte une quatrième languette qui fait saillie d'une quatrième portion du bord supérieur, préférentiellement en vis-à-vis de la première portion.

Préférentiellement, les moyens de serrage des languettes se présentent sous la forme d'un ou de plusieurs liens s'étendant entre des moyens de fixations répartis sur lesdites languettes, lesdits moyens de fixation comportant des moyens d'enroulement desdits liens.
[Fig. 1] est une vue de profil d'un dispositif selon l'invention,
[Fig. 2] est une vue d'une partie d'un dispositif selon l'invention, installé sur la partie restante d'un membre amputé d'un patient,
[Fig. 3] est une vue d'une partie d'un dispositif selon l'invention, installé sur la partie restante d'un membre amputé d'un patient,

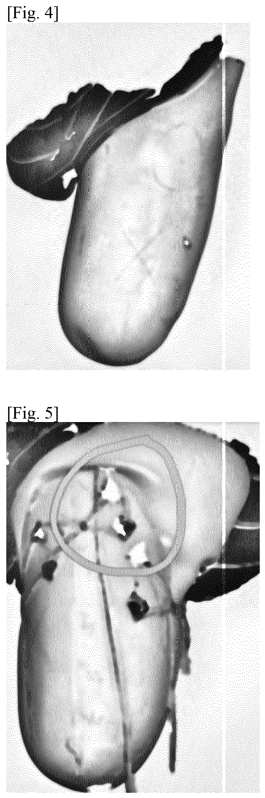

[Fig. 4] est une image d'un premier modèle numérique 3D obtenu dans la méthode selon l'invention,
[Fig. 5] est une image d'un second modèle numérique 3D, obtenu dans la méthode selon l'invention,

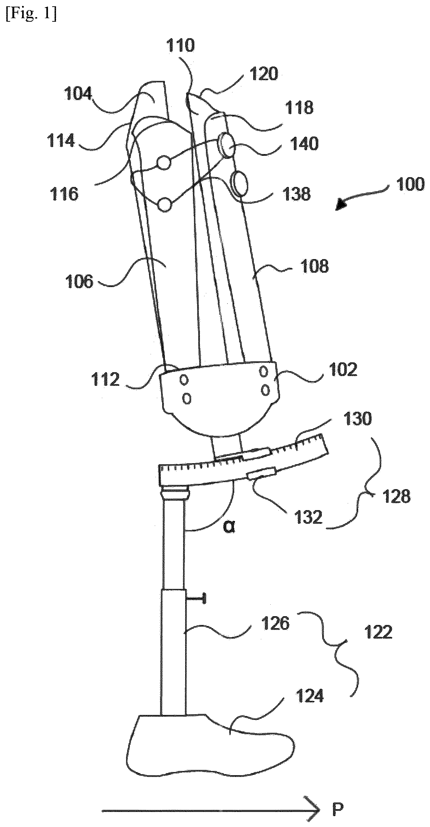

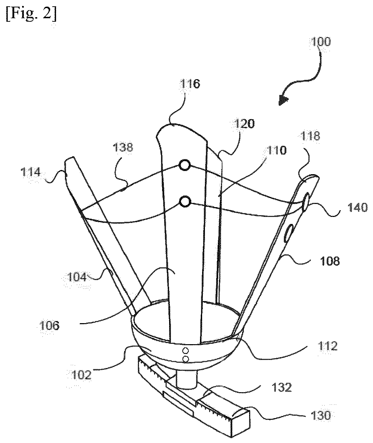

Les Figs. 1 et 2 montrent un dispositif 100, selon l'invention, d'aide à la prise d'empreintes par numérisation de la partie restante d'un membre amputé d'un patient pour la génération d'un modèle 3D d'une emboîture de prothèse fémorale.

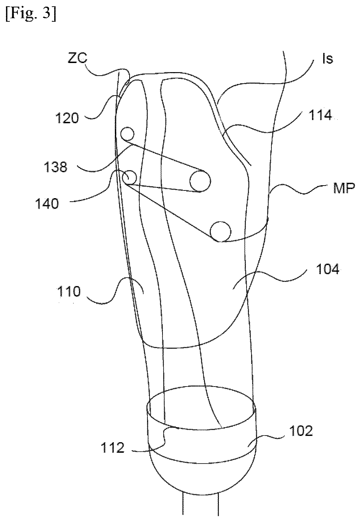

Sur la Fig.3, le dispositif 100 illustré à la Fig. 1 est installé sur la partie restante du membre amputé d'un patient en vue d'être scanné par le scanner 3-D. Par la suite, la « partie restante du membre amputé » sera nommé « membre ».

Le dispositif 100 comprend une base 102 et des languettes 104, 106, 108, 110, solidaires de la base 102, en matériau transparent.

La base 102 sert de support aux languettes et prend ici la forme d'une coupelle. Elle présente un bord supérieur 112, circulaire.

La première languette 104 fait saillie de la base 102 depuis une première portion du bord supérieur circulaire 112 qui correspond à un premier quart de ce bord. Cette première languette est destinée à être appliquée contre la partie du membre du patient qui contient l'ischion et est utilisée pour repérer la position de l'ischion. Elle présente à son extrémité libre un bord supérieur incliné 114 en direction de la base 102 et destiné à être appliqué contre le bord antérieur de l'ischion. De manière à optimiser son application contre le membre, la face interne de la languette 104 se trouve sensiblement concave.

La seconde languette 106 fait saillie depuis le quart adjacent au premier quart du bord supérieur 112 de la base 102. Elle est destinée à être appliquée contre la portion arrière de l'entrecuisse et présente un bord supérieur 116 qui s'évase en direction de l'extérieur de manière à épouser le haut de l'entrecuisse. De manière à optimiser son application contre le membre, la face interne de la languette 106 se trouve aussi sensiblement concave.

La troisième languette 108 fait saillie depuis le quart en vis-à-vis du premier quart du bord supérieur 112 de la base 102. Elle est destinée à être appliquée contre une portion avant de l'entrecuisse et présente un bord supérieur 118 incliné en direction de la base et sensiblement conforme au pli de l'aine. De manière à optimiser son application contre le membre, la face interne de la languette 108 se trouve aussi sensiblement concave.

La quatrième languette 110 fait saillie depuis l'autre quart adjacent au premier quart du bord supérieur 112 de la base 102. Elle est destinée à être appliquée contre la face externe du membre, la face incluant le trochanter supérieur, et présente un bord supérieur 120 incliné en direction de la base 102. De manière à optimiser son application contre le membre, la face interne de la languette 110 se trouve aussi sensiblement concave.

Il convient de noter que la détermination des formes respectives des languettes est facilement appréciable par un homme du métier, qui est ici un orthoprothésiste et qui saura adapter ces formes aux régions du membre contre lesquelles elles s'appliquent. Par ailleurs, des dispositifs 100 pourront être conçus sans difficulté, les uns pour des membres droits, les autres pour des membres gauches.

Pour que les prises d'empreintes soient plus précises et reproduisent au mieux les conditions dans lesquelles le membre sera chaussé de l'emboîture de sa future prothèse, le dispositif 100 comprend un appui au sol 122 pour placer le patient en position debout une fois que le dispositif 100 est installé sur le membre.

L'appui au sol 122 est composé d'un piètement 124, qui prend avantageusement la forme d'un pied humain, et d'un mât tubulaire 126 réglable en hauteur. La configuration en pied humain du piètement et le réglage de la hauteur du mât tubulaire 126 permettent de positionner le patient en position debout et stable lors de la prise d'empreintes. En particulier, le réglage en hauteur du mât 126 permet de rendre le dispositif 100 utilisable chez tout patient.

L'appui au sol 122 est relié à la base 102 par l'intermédiaire d'un moyen de liaison 128. Pour respecter le flexum de la hanche du patient en position stable debout, ce moyen de liaison 128 permet ce réglage du flexum et comporte, pour ce faire, des moyens de réglage de la position relative des languettes 104, 106, 108, 110 solidaires de la base 102 par rapport à l'appui au sol 122, et de l'angle antéro postérieur formé entre eux. Les moyens de réglage comprennent une glissière 130 concave, dont la concavité est tournée vers la base 102, ainsi qu'un guide 132 que présente la base 102. Le guide 132 peut coulisser le long de la glissière 130 dans le sens antéro-postérieur P.

Pour faciliter l'utilisation du dispositif 100 une fois chaussé sur le membre et notamment permettre au patient de s'assoir si besoin, une liaison articulée 134, telle qu'un pivot, peut en outre être prévue sur le mât tubulaire 126, préférentiellement à proximité du moyen de liaison 128 (fig.2).

Le dispositif 100 comporte encore des moyens de serrage des languettes 104, 106, 108, 110 contre le membre. Ils se présentent sous la forme de liens 138 s'étendant entre des moyens de fixations 140 répartis sur les languettes. Les moyens de fixation comportent des moyens d'enroulement des liens 138 pour assurer le serrage.

Le dispositif 100 est donc utilisé pour la prise d'empreintes par numérisation dans une méthode de génération d'un modèle 3D d'une emboîture de prothèse fémorale pour un patient en vue de sa conception assistée par ordinateur. Les étapes de la méthode selon l'invention sont détaillées dans ce qui suit.

Dans une première étape, préalablement à la mise en place du dispositif 100 sur le membre, des points de repères sont effectués sur celui-ci, tels que des points ou des croix, par exemple à l'aide d'un marqueur. Dans une seconde étape, le membre est numérisé à l'aide d'un appareil adapté. Un premier modèle numérique 3D est ainsi obtenu, sur lequel sont visibles les contours du membre.

A l'étape suivante, le dispositif 100 est installé sur le membre MP (figure 3). La mise en place du dispositif 100 sur le membre MP, telle qu'illustrée sur la figure 3, est extrêmement simple et rapide. Le patient est positionné en position debout et le membre MP est reçu dans l'espace délimité par les languettes 104, 106, 108, 110. Le bord supérieur 114 de la première languette 104 est appliqué contre le bord antérieur de l'ischion. Les seconde et troisième languettes 106 et 108 sont appliquées respectivement contre les portions arrière et avant de la zone d'entrecuisses du membre MP. Le bord supérieur 116 de la seconde languette 106 est disposé au niveau du haut de l'entrecuisse. Le bord supérieur 118 de la troisième languette est appliqué contre le pli de l'aine.

Une fois installées, les languettes sont serrées contre le membre MP grâce aux moyens de serrage 136, de manière à comprimer les masses molles.

La stabilité et le flexum de la hanche du patient sont réglés par coulissement de la glissière 130 sur le guide 132.

Une fois le serrage et les autres réglages réalisés, les zones de compression ZC des masses molles et la position de l'ischion Is sont visibles sur le membre MP (figure 3). A l'étape suivante, le membre ainsi chaussé du dispositif 100 est numérisé et un second modèle numérique 3D est ainsi obtenu, sur lequel sont visibles les zones de compression des masses molles, la position de l'ischion et les points de repères réalisés sur le membre.

A l'étape suivante, les premier et second modèles numériques 3D obtenus sont superposés par alignement des points de repères de chaque modèle numérique 3D. Sur le second modèle numérique 3D, seule la partie haute sur laquelle sont visibles le bord antérieur de l'ischion et les zones de compression des masses molles est conservée. Cela correspond environ aux dix premiers centimètres, en partant du haut du modèle. La partie basse restante est éliminée. On obtient alors un second modèle numérique 3D tronché.

Sur le premier modèle numérique 3D, seule est conservée la partie basse nécessaire à la reconstitution, à partir du second modèle numérique 3D tronché, d'un modèle numérique 3D entier. La partie restante est éliminée.

Le modèle numérique 3D ainsi généré est apte à être utilisé pour la conception assistée par ordinateur.

## Revendications

1. Dispositif (100) d'aide à la prise d'empreintes par numérisation de la partie restante d'un membre amputé (MP) d'un patient pour la génération d'un modèle 3D d'une emboîture de prothèse fémorale, **caractérisé en ce qu'**il comprend :
- une base (102),
- au moins deux languettes (104 ; 106) transparentes solidaires de la base (102) et aptes à être appliquées contre la partie restante du membre amputé (MP),
- des moyens de serrage desdites languettes (104 ; 106) sur la partie restante du membre amputé (MP).

2. Dispositif selon la revendication 1, **caractérisé en ce qu'**il comprend, de plus :
- un appui au sol (122)
- un moyen de liaison (128) entre ledit appui au sol (122) et ladite base (102).

3. Dispositif selon la revendication 2, **caractérisé en ce que** ledit moyen de liaison (128) comporte des moyens de réglage de la position relative desdites languettes (104 ; 106), solidaires de la base (102), par rapport audit appui au sol (122) ainsi que de l'angle antéro postérieur formé entre eux.

4. Dispositif selon la revendication 3, **caractérisé en ce que** lesdits moyens de réglage comprennent une glissière (130) concave dont la concavité est tournée vers la base (102) ainsi qu'un guide (132) que présente la base (102), ledit guide (132) étant apte à coulisser le long de ladite glissière (130).

5. Dispositif selon l'une des revendications 2 à 4, **caractérisé en ce que** ledit appui au sol (122) comporte un piètement (124) et un mât (126) réglable en hauteur.

6. Dispositif selon la revendication 5, **caractérisé en ce que** ledit piètement (124) prend la forme d'un pied humain.

7. Dispositif selon l'une des revendications 2 à 6, **caractérisé en ce que** ledit appui au sol (122) comprend, de plus, une liaison articulée, préférentiellement un pivot.

8. Dispositif (100) selon l'une des revendications précédentes, **caractérisé en ce que** la première des languettes (104) présente un bord supérieur (114) incliné en direction de la base (102).

9. Dispositif selon l'une des revendications précédentes, **caractérisé en ce que** la première languette (104) fait saillie depuis une première portion du bord supérieur (112) que présente la base (102), la seconde languette (106) fait saillie depuis une seconde portion du bord supérieur (112) adjacente à la première portion.

10. Dispositif selon l'une des revendications précédentes, **caractérisé en ce qu'**il comporte une troisième languette (108) qui fait saillie d'une troisième portion du bord supérieur (112) de ladite base (102).

11. Dispositif selon la revendication 10, **caractérisé en ce qu'**il comporte une quatrième languette (110) qui fait saillie d'une quatrième portion du bord supérieur (112) de ladite base (102).

12. Dispositif selon l'une des revendications précédentes, **caractérisé en ce que** les moyens de serrage des languettes se présentent sous la forme d'un ou de plusieurs liens (138) s'étendant entre des moyens de fixations (140) répartis sur lesdites languettes (104 ; 106), lesdits moyens de fixation (140) comportant des moyens d'enroulement desdits liens (138).

13. Méthode de génération d'un modèle 3D d'une emboîture de prothèse fémorale pour un patient en vue de sa conception assistée par ordinateur, la méthode comprenant une étape de prises d'empreintes par numérisation comprenant les étapes suivantes :
- marquage d'au moins un point de repère sur la partie restante d'un membre amputé (MP),
- numérisation 3D de la partie restante du membre amputé (MP) à l'aide d'un scanner et obtention d'un premier modèle numérique,
- installation, sur la partie restante du membre amputé, d'un dispositif (100) comportant une base (102), au moins deux languettes (104 ; 106) transparentes solidaires de la base et des moyens de serrage des languettes sur la partie restante du membre amputé (MP),
- mise en place dudit dispositif (100) par mise en place du bord supérieur (114) d'une des languettes (104) au niveau du bord antérieur de l'ischion et obtention de la position de l'ischion (Is),
- serrage des moyens de serrage pour comprimer les masses molles de la partie restante du membre amputé (MP) avec les languettes (104 ; 106), et obtention de zones de compression des masses molles (ZC),
- numérisation 3D de la partie restante du membre amputé (MP) chaussé du dispositif (100) et obtention d'un second modèle numérique,
- superposition des premier et second modèles numériques 3D obtenus par alignement des points de repère de chaque modèle numérique,
- conservation sur le second modèle numérique 3D, de la partie haute sur laquelle sont visibles le bord antérieur de l'ischion (Is) et les zones de compression des masses molles (ZC), élimination de la partie basse restante et obtention d'un second modèle numérique 3D tronché,
- conservation sur le premier modèle numérique 3D, de la partie basse permettant la reconstitution, à partir du second modèle numérique 3D tronché, d'un modèle numérique 3 D entier, et élimination de la partie restante,
- génération d'un modèle 3D.

14. Méthode selon la revendication 13, **caractérisée en ce que** ledit dispositif (100) comportant de plus, un appui au sol (122) et des moyens de réglage (130 ; 132) de la position relative desdites languettes (104 ; 106) solidaires de la base (102) par rapport audit appui au sol, ainsi que de l'angle antéro-postérieur formé entre eux, la méthode comprend :
- une étape de réglage de flexum de la hanche dudit patient.

## Patentansprüche

1. Vorrichtung (100) zur Unterstützung beim Nehmen von Abdrücken durch Digitalisierung des verbleibenden Teils einer amputierten Gliedmaße (MP) eines Patienten zur Erzeugung eines 3D-Modells eines Oberschenkelprothesenschafts, **dadurch gekennzeichnet, dass** sie umfasst:
- eine Basis (102),
- mindestens zwei transparente Zungen (104; 106), die fest mit der Basis (102) verbunden sind und geeignet sind, an den verbleibenden Teil der amputierten Gliedmaße (MP) angelegt zu werden,
- Mittel zum Spannen der Zungen (104; 106) auf dem verbleibenden Teil der amputierten Gliedmaße (MP).

2. Vorrichtung nach Anspruch 1, **dadurch gekennzeichnet, dass** sie ferner umfasst:
- eine Bodenabstützung (122)
- ein Verbindungsmittel (128) zwischen der Bodenabstützung (122) und der Basis (102).

3. Vorrichtung nach Anspruch 2, **dadurch gekennzeichnet, dass** das Verbindungsmittel (128) fest mit der Basis (102) verbundene Mittel zum Einstellen der relativen Position der Zungen (104; 106) in Bezug auf die Bodenabstützung (122) sowie des zwischen ihnen gebildeten anteroposterioren Winkels beinhaltet.

4. Vorrichtung nach Anspruch 3, **dadurch gekennzeichnet, dass** die Mittel zum Einstellen eine konkave Schiene (130) umfassen, deren Konkavität zu der Basis (102) hin gewandt ist, sowie eine Führung (132), welche die Basis (102) aufweist, wobei die Führung (132) geeignet ist, entlang der Schiene (130) zu gleiten.

5. Vorrichtung nach einem der Ansprüche 2 bis 4, **dadurch gekennzeichnet, dass** die Bodenabstützung (122) ein Fußteil (124) und einen höhenverstellbaren Schaft (126) beinhaltet.

6. Vorrichtung nach Anspruch 5, **dadurch gekennzeichnet, dass** das Fußteil (124) die Form eines menschlichen Fußes annimmt.

7. Vorrichtung nach einem der Ansprüche 2 bis 6, **dadurch gekennzeichnet, dass** die Bodenabstützung (122) ferner eine Gelenkverbindung, bevorzugt einen Drehzapfen, umfasst.

8. Vorrichtung (100) nach einem der vorhergehenden Ansprüche, **dadurch gekennzeichnet, dass** die erste der Zungen (104) einen oberen Rand (114) aufweist, der in Richtung der Basis (102) geneigt ist.

9. Vorrichtung nach einem der vorhergehenden Ansprüche, **dadurch gekennzeichnet, dass** die erste Zunge (104) von einem ersten Abschnitt des oberen Rands (112), den die Basis (102) aufweist, absteht, wobei die zweite Zunge (106) von einem zweiten Abschnitt des oberen Rands (112), der zu dem ersten Abschnitt benachbart ist, absteht.

10. Vorrichtung nach einem der vorhergehenden Ansprüche, **dadurch gekennzeichnet, dass** sie eine dritte Zunge (108) beinhaltet, die von einem dritten Abschnitt des oberen Rands (112) der Basis (102) absteht.

11. Vorrichtung nach Anspruch 10, **dadurch gekennzeichnet, dass** sie eine vierte Zunge (110) beinhaltet, die von einem vierten Abschnitt des oberen Rands (112) der Basis (102) absteht.

12. Vorrichtung nach einem der vorhergehenden Ansprüche, **dadurch gekennzeichnet, dass** die Mittel zum Spannen der Zungen in Form einer oder mehrerer Schnüre (138) vorliegen, die sich zwischen Befestigungsmitteln (140) erstrecken, die auf den Zungen (104; 106) verteilt sind, wobei die Befestigungsmittel (140) Mittel zum Aufrollen der Schnüre (138) beinhalten.

13. Verfahren zur Erzeugung eines 3D-Modells eines Oberschenkelprothesenschafts für einen Patienten im Hinblick auf dessen computergestützten Entwurf, wobei das Verfahren einen Schritt des Nehmens von Abdrücken durch Digitalisierung umfasst, der die folgenden Schritte umfasst:
- Kennzeichnen mindestens eines Markierungspunkts auf dem verbleibenden Teil einer amputierten Gliedmaße (MP),
- 3D-Digitalisierung des verbleibenden Teils der amputierten Gliedmaße (MP) mithilfe eines Scanners und Erhalten eines ersten digitalen Modells,
- Anbringen, an dem verbleibenden Teil der amputierten Gliedmaße, einer Vorrichtung (100), die eine Basis (102), mindestens zwei fest mit der Basis verbundene transparente Zungen (104; 106) und Mittel zum Spannen der Zungen auf dem verbleibenden Teil der amputierten Gliedmaße (MP) beinhaltet,
- Einrichten der Vorrichtung (100) durch Einrichten des oberen Rands (114) einer der Zungen (104) an dem vorderen Rand des Sitzbeins und Erhalten der Position des Sitzbeins (Is),
- Spannen der Spannmittel, um die Weichteile des verbleibenden Teils der amputierten Gliedmaße (MP) mit den Zungen (104; 106) zu komprimieren, und Erhalten von Kompressionszonen der Weichteile (ZC),
- 3D-Digitalisierung des verbleibenden Teils der amputierten Gliedmaße (MP), auf welche die Vorrichtung (100) aufgezogen ist, und Erhalten eines zweiten digitalen Modells,
- Überlagern der ersten und zweiten erhaltenen digitalen 3D-Modelle durch Ausrichten der Markierungspunkte jedes digitalen Modells,
- Beibehalten auf dem zweiten digitalen 3D-Modell des oberen Teils, auf dem der vordere Rand des Sitzbeins (Is) und die Kompressionszonen der Weichteile (ZC) sichtbar sind, Entfernen des verbleibenden unteren Teils und Erhalten eines gekappten zweiten digitalen 3D-Modells,
- Beibehalten auf dem ersten digitalen 3D-Modell des unteren Teils, was die Rekonstruktion, ausgehend von dem gekappten zweiten digitalen 3D-Modell, eines vollständigen digitalen 3D-Modells ermöglicht, und Entfernen des verbleibenden Teils,
- Erzeugen eines 3D-Modells.

14. Verfahren nach Anspruch 13, **dadurch gekennzeichnet, dass** die Vorrichtung (100) ferner eine Bodenabstützung (122) und Mittel zum Einstellen (130; 132) der relativen Position der fest mit der Basis (102) verbundenen Zungen (104; 106) in Bezug auf die Bodenabstützung sowie des zwischen ihnen gebildeten anteroposterioren Winkels beinhaltet, wobei das Verfahren umfasst:
- einen Schritt des Einstellens der Beugestellung der Hüfte des Patienten.

## Claims

1. Device (100) for assistance in acquiring digitized impressions of the remaining part of an amputated limb (MP) of a patient in order to generate a 3D model of a femoral prosthesis socket, **characterized in that** it comprises:
- a base (102),
- at least two transparent tongues (104; 106) integral with the base (102) and able to be applied against the remaining part of the amputated limb (MP),
- tightening means for tightening said tongues (104; 106) on the remaining part of the amputated limb (MP).

2. Device according to Claim 1, **characterized in that** it further comprises:
- a ground support (122),
- a connecting means (128) between said ground support (122) and said base (102).

3. Device according to Claim 2, **characterized in that** said connecting means (128) comprises adjustment means for adjusting the relative position of said tongues (104; 106), integral with the base (102), with respect to said ground support (122), and also for adjusting the antero-posterior angle formed between them.

4. Device according to Claim 3, **characterized in that** said adjustment means comprise a concave slide (130), the concavity of which faces the base (102), and a guide (132) provided on the base (102), said guide (132) being able to slide along said slide (130).

5. Device according to any one of Claims 2 to 4, **characterized in that** said ground support (122) comprises a bottom part (124) and a pole (126), which is adjustable in height.

6. Device according to Claim 5, **characterized in that** said bottom part (124) takes the form of a human foot.

7. Device according to any one of Claims 2 to 6, **characterized in that** said ground support (122) further comprises an articulated connection, preferably a pivot.

8. Device (100) according to any one of the preceding claims, **characterized in that** the first of the tongues (104) has an upper edge (114) inclined in the direction of the base (102).

9. Device according to any one of the preceding claims, **characterized in that** the first tongue (104) projects from a first portion of the upper edge (112) of the base (102), and the second tongue (106) projects from a second portion of the upper edge (112) adjacent to the first portion.

10. Device according to any one of the preceding claims, **characterized in that** it comprises a third tongue (108), which projects from a third portion of the upper edge (112) of said base (102).

11. Device according to Claim 10, **characterized in that** it comprises a fourth tongue (110), which projects from a fourth portion of the upper edge (112) of said base (102).

12. Device according to any one of the preceding claims, **characterized in that** the means for tightening the tongues are in the form of one or more ties (138) extending between fastening means (140) distributed on said tongues (104; 106), said fastening means (140) having means for winding said ties (138).

13. Method for generating a 3D model of a femoral prosthesis socket for a patient with a view to its computer-aided design, the method comprising a step of acquiring impressions by digitization comprising the following steps:
- marking of at least one reference point on the remaining part of an amputated limb (MP),
- 3D digitization of the remaining part of the amputated limb (MP) with the aid of a scanner, and obtaining a first digital model,
- installation, on the remaining part of the amputated limb, of a device (100) having a base (102), at least two transparent tongues (104; 106) integral with the base, and tightening means for tightening the tongues on the remaining part of the amputated limb (MP),
- positioning of said device (100) by positioning the upper edge (114) of one of the tongues (104) at the level of the anterior margin of the ischium, and obtaining the position of the ischium (Is),
- tightening of the tightening means in order to compress the soft tissues of the remaining part of the amputated limb (MP) with the tongues (104; 106), and obtaining soft-tissue compression zones (ZC),
- 3D digitization of the remaining part of the amputated limb (MP) fitted with the device (100), and obtaining a second digital model,
- superpositioning of the first and second 3D digital models obtained, by aligning the reference points of each digital model,
- preservation, on the second digital model 3D, of the upper part on which the anterior margin of the ischium (Is) and the soft-tissue compression zones (ZC) are visible, elimination of the remaining lower part, and obtaining a second, 3D truncated digital model,
- preservation, on the first 3D digital model, of the lower part allowing the reconstruction, from the second, truncated 3D digital model, of an entire 3D digital model, and elimination of the remaining part,
- generation of a 3D model.

14. Method according to Claim 13, **characterized in that**, with said device (100) further comprising a ground support (122) and adjustment means (130; 132) for adjusting the relative position of said tongues (104; 106), integral with the base (102), with respect to said ground support, and also for adjusting the antero-posterior angle formed between them, the method comprises:
- a step of adjusting the flexum of the hip of said patient.
